# EUROPEAN PATENT APPLICATION

(11) **EP 4 813 292 A1**
(43) Date of publication of application: **30.09.2026**
(21) Application number: 25166557.6
(22) Date of filing: 27.03.2025
(51) Int. Cl.: A61B 5/113

(54) **A SENSOR SYSTEM**

(71) Applicant: Koninklijke Philips N.V., 5656 AE Eindhoven (NL)
(72) Inventor: WIEMKER, Rafael, Eindhoven (NL); FRERKING, Lena Christina, Eindhoven (NL); VAN EE, Raymond, 5656AG Eindhoven (NL); NAUTS, Sanne, Eindhoven (NL); HELLE, Michael Günter, Eindhoven (NL); WEIß, Steffen, Eindhoven (NL)
(74) Representative: Philips Intellectual Property & Standards

(57) **Abstract**

The present disclosure provides concepts for determining cardiac and/or respiratory information of a subject. To this end, a cushion is provided that is configured for compression by movement of at least a part of a torso of a subject, the cushion containing a fluid, wherein a fluid pressure within the cushion changes responsive to compression of the cushion. Furthermore, an amplification arrangement is provided that has a visual characteristic dependent upon the fluid pressure within the cushion. The visual characteristic of the amplification arrangement over a time period is therefore recorded (e.g., by a camera), and the recorded visual characteristic is analyzed to generate cardiac information and/or respiratory information of the subject during the time period.

## Description

### FIELD OF INVENTION

The present invention relates to sensor systems for monitoring physiological signals, and in particular, to systems and methods for detecting cardiac and/or respiratory signals using a fluid-filled cushion.

### BACKGROUND OF THE INVENTION

During medical imaging procedures such as MRI or CT scans, monitoring a subject's physiological signals is crucial for ensuring image quality and subject safety. For example, it may be desired to track the subject's respiratory signal in order to ascertain the timing of breath holds, and therefore determine when image acquisition is of high quality. It may also be necessary to track cardiac signals to acquire desired cardiac images, and to determine when the subject is at their stillest.

Respiratory bellows are commonly used to track breathing patterns by detecting pressure changes as the subject's chest moves. These air-filled cushions provide valuable respiratory data but require wires to transmit the respiratory signal. Said wires may be a nuisance to set up and may obstruct natural movement of a caregiver and/or subject.

Furthermore, camera-based methods have recently been utilized to directly observe the breathing of the subject and therefore deduce a respiratory signal. Furthermore, it has also been proposed to detect subtle skin color changes via cameras to measure cardiac signals. Whilst these approaches provide a contact and lead-less way to measure physiological signals, they also face limitations - they require an unobstructed view of the subject's skin, can be affected by factors like makeup or facial hair, and may not work well for all skin tones.

Accordingly, there exists a need for an improved means for measuring cardiac and/or respiratory signals that may overcome one or more of these problems.

### SUMMARY OF INVENTION

The invention is defined by the claims.

According to an aspect of the present disclosure, a sensor system is provided. The sensor system includes a cushion configured for compression by movement of at least a part of a torso of a subject. The cushion contains a fluid, and a fluid pressure within the cushion changes responsive to compression of the cushion. The system further includes an amplification arrangement having a visual characteristic dependent upon the fluid pressure within the cushion, a camera configured to record the visual characteristic of the amplification arrangement over a time period, and a processor configured to analyse the recorded visual characteristic. The processor is further configured to generate cardiac information and/or respiratory information of the subject during the time period based on the analysis.

This sensor system allows for non-invasive monitoring of cardiac and/or respiratory information without the need for additional sensor wiring, reducing setup complexity and subject discomfort while providing valuable physiological data.

To this end, proposed embodiments utilize an amplification arrangement that translates small pressure variations of fluid (e.g., air, liquid) within the cushion into visible changes in a visual characteristic. For example, this visual characteristic may be a color change in a pressure-sensitive fluid, the movement of a mechanical structure, or alterations in light wave guidance properties. A camera records these visual changes over time, allowing a processor to analyze the data and generate cardiac and/or respiratory information.

It is established that pressure variations of fluid within a cushion compressible by movement of a torso of a subject can be utilized to generate a respiration information. Nevertheless, this information is typically measured electronically and transmitted via cables. Thus, it is proposed to provide a means of amplifying and presenting this pressure change information visually, such that a camera can directly observe the amplified pressure changes and thus determine the respiratory information in a remote manner.

Furthermore, it has been realized that cardiac movement (e.g., due to expansion and contraction of blood vessels near the skin surface of the subject) may also cause a pressure variation of fluid within a compressible cushion. Whilst this variation is small compared to the respiratory movement, it is still noticeable. Therefore, by amplifying and presenting this pressure change information visually, the cardiac information may also be derived from pressure changes observed by a camera. This may be an invaluable way of producing cardiac information of the subject that avoids the need for cables, in contrast to traditional methods using ECG sensors or pulse oximeters.

This innovative approach thus combines the simplicity of cushions used for deriving respiratory information (such as traditional respiratory bellows) with optical sensing techniques, in such a way that obviates the need for cables. Furthermore, by facilitating the generation of both respiratory and cardiac information from a single device, the system reduces setup complexity and subject discomfort while providing valuable physiological data.

The proposed embodiment offers several advantages over existing monitoring methods. It eliminates the need for separate ECG leads or pulse oximeters, reducing signal cables and distinct power supplies that can complicate scan preparation and operation. The system can complement or enhance camera-based vital sign detection methods, particularly in situations where direct skin visibility is limited or compromised.

Furthermore, the sensor system can be integrated with existing medical imaging equipment, such as MRI coils, streamlining the overall imaging process. This versatility makes the invention suitable for a wide range of clinical and research applications, potentially improving image quality and subject monitoring capabilities across various medical imaging modalities.

In a first embodiment, the amplification arrangement may comprise a pressure sensitive fluid having a colour characteristic dependent upon the fluid pressure within the cushion, and the camera may be configured to record the colour characteristic of the pressure sensitive fluid over the time period.

Using a pressure sensitive fluid with a color characteristic provides a clear visual indicator of pressure changes, enhancing the system's sensitivity to subtle cardiac and respiratory movements. Pressure sensitive fluid may be relatively inexpensive and simple to integrate visual means of pressure fluctuation indication.

In another embodiment, the amplification arrangement may comprise a mechanical structure having a position and/or shape dependent upon the fluid pressure within the cushion, and the camera may be configured to record the position and/or shape of the mechanical structure over the time period.

A mechanical structure offers a robust and potentially more precise method of visualizing pressure changes, which can improve the accuracy of the derived cardiac and respiratory information.

In a further embodiment, the amplification arrangement may comprise an optical structure having a light wave guidance property dependent upon the fluid pressure within the cushion; a light source configured to provide light to the optical structure; and wherein the camera may be configured to record at least a part of the path of the light provided by the light source.

An optical structure utilizing light wave guidance properties can provide highly sensitive pressure measurements, potentially capturing even the most subtle cardiac signals.

In yet another embodiment, the amplification arrangement may comprise a reflective surface having a position and/or orientation dependent upon the fluid pressure within the cushion; a directional light source configured to provide a beam of light incident to the reflective surface; and wherein the camera may be configured to record a resulting direction of the beam of light reflected by the reflective surface.

This configuration using a reflective surface and directional light source can amplify small pressure changes into larger, more easily detectable light beam movements, enhancing the system's sensitivity.

Of course, it should be noted that a combination of these potential amplification arrangements (and multiple of each amplification arrangement) may be utilized to provide redundancy (in case the means are visually obstructed) and improve accuracy and reliability of the system.

The sensor system may further comprise a biasing means configured to pressurize the cushion toward the torso of the subject. For instance, the biasing means may comprise a magnetic resonance (MR) coil. In this case, the amplification arrangement may be at least partially integrated with the MR coil.

A biasing means ensures consistent contact between the cushion and the subject's torso, improving signal quality and reliability of the measurements. Integration with an MR coil allows for seamless incorporation of the sensor system into existing MRI setups, reducing additional equipment and simplifying the overall imaging process.

The cushion may further contain a compressible solid medium.

Including a compressible solid medium can help optimize the cushion's response to pressure changes, potentially improving the system's sensitivity to both cardiac and respiratory movements.

The cushion may be configured to be positioned proximate to the heart of the subject.

Positioning the cushion near the heart maximizes the capture of cardiac information, potentially improving the accuracy and reliability of the cardiac information generated. That is, the cushion can be positioned near the heart to maximize cardiac information capture, potentially improving the accuracy and reliability of the generated information.

The processor may be configured to analyse the recorded visual characteristic to determine pressure changes within the cushion over the time period; and generate the cardiac information and/or the respiratory information based on the determined pressure changes.

This configuration allows for direct translation of visual data into meaningful physiological information, streamlining the data processing workflow.

The processor may be configured to receive external respiratory information from a further sensor, the external respiratory information describing respiration of the subject during the time period; analyse the recorded visual characteristic and the respiratory information, and to generate, based on the analysis, the cardiac information.

By incorporating external respiratory information, the system can more accurately isolate and analyze the cardiac information, potentially improving the quality of the cardiac information generated. Indeed, the respiratory information may be more easily derived via other means, and so these other means may be utilized to improve accuracy of cardiac information generation.

The cardiac information may comprise a cardiac signal, a heart rate, and/or a heart rate variability, and wherein the respiratory information may comprise a respiratory signal, a respiratory rate and/or breath hold timings.

That is, in some embodiments it may only be desired to obtain high-level information, such as heart rate or respiratory rate. In other cases, it may be desired to capture the full cardiac signal or respiratory signal to understand the exact state of the subject at a given moment in time. Providing a range of cardiac and respiratory metrics thus enhances the utility of the system for various clinical and research applications.

According to a further aspect of the present disclosure, a method for generating cardiac information and/or respiratory information of a subject is provided.

The method comprises compressing a cushion by movement of at least a part of a torso of a subject, wherein fluid pressure within the cushion changes responsive to compression of the cushion; recording a visual characteristic of an amplification arrangement over a time period, wherein the visual characteristic of the amplification arrangement is dependent upon the fluid pressure within the cushion; and analysing the recorded visual characteristic to generate cardiac information and/or respiratory information of the subject during the time period.

According to another aspect of the present disclosure, a computer program comprising computer program code means adapted, when said computer program is run on a computer, to implement the method of the second aspect is provided.

According to a final aspect of the present disclosure, an apparatus adapted to provide an indicator of cardiac information and/or respiratory information of a subject is provided. The apparatus comprises a cushion configured for compression by movement of at least a part of a torso of a subject, the cushion containing a fluid, and wherein a fluid pressure within the cushion changes responsive to compression of the cushion; and an amplification arrangement having a visual characteristic dependent upon the fluid pressure within the cushion.

This apparatus offers a simplified, standalone solution for monitoring cardiac and/or respiratory information, potentially useful in a variety of clinical and research settings where more complex monitoring systems may not be practical or available. Indeed, such a system facilitates the determination of respiratory and/or cardiac related movement by a camera system, for instance, or by an observer.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a block diagram of an apparatus adapted to provide an indicator of cardiac information and/or respiratory information of a subject according to aspects of the present disclosure;
Fig. 2 illustrates a simplified view of a potential implementation of the apparatus according to an aspect of an embodiment;
Fig. 3 illustrates a simplified view of a potential implementation of the apparatus according to a further embodiment;
Fig. 4 illustrates a block diagram of a sensor system according to an embodiment.
Fig. 5 illustrates a flowchart depicting a method for generating cardiac and/or respiratory information, according to aspects of the present disclosure; and
Fig. 6 is a simplified block diagram of a computer within which one or more parts of an embodiment may be employed.

### DETAILED DESCRIPTION OF EMBODIMENTS

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides concepts for sensing cardiac and/or respiratory information of a subject. Specifically, a cushion is utilized which may be positioned over at least part of a torso of the subject, and which compresses responsive to movement of the torso. As a result of the compression, a fluid pressure within the cushion changes. To utilize this aspect of the cushion, an amplification arrangement having a visual characteristic dependent upon the fluid pressure within the cushion is provided. Accordingly, as the torso of the subject moves (e.g., due to respiratory and/or cardiac motion), the visual characteristic changes by virtue of the change in fluid pressure within the cushion. This visual characteristic may be recorded by a camera over time, with the recording leveraged to generate cardiac and/or respiratory information (e.g., a heart rate, respiratory rate, cardiac signal and/or respiratory signal).

In other words, the present disclosure provides concepts for determining cardiac and/or respiratory information of a subject. To this end, a cushion is provided that is configured for compression by movement of at least a part of a torso of a subject, the cushion containing a fluid, wherein a fluid pressure within the cushion changes responsive to compression of the cushion. Furthermore, an amplification arrangement is provided that has a visual characteristic dependent upon the fluid pressure within the cushion. The visual characteristic of the amplification arrangement over a time period is therefore recorded (e.g., by a camera), and the recorded visual characteristic is analyzed to generate cardiac information and/or respiratory information of the subject during the time period.

The disclosed concepts therefore provide a means for measuring cardiac and/or respiratory information of a subject within the need for electronic components and cables leading from the subject. This may help to improve ease of setup of an examination procedure, comfort of the subject, and alleviate problems related to wiring obstructing movement of caregivers.

In medical imaging procedures such as magnetic resonance (MR) or computed tomography (CT) scans, monitoring cardiac and respiratory motion of the subject may be crucial for optimizing image quality and ensuring subject safety. Conventional methods for capturing cardiac and respiratory signals often involve the use of multiple sensors, which can be cumbersome to set up and may introduce additional wiring into the subject environment. These traditional approaches can lead to increased preparation time, subject discomfort, and potential interference with imaging equipment.

The proposed sensor system addresses these challenges by providing a simplified and efficient method for capturing cardiac and respiratory information. The system utilizes a cushion containing a fluid, which is configured to be compressed by the movement of a subject's torso. As the subject breathes or as their heart beats, the fluid pressure within the cushion changes in response to these physiological movements.

An amplification arrangement is incorporated into the system, featuring a visual characteristic that varies depending on the fluid pressure within the cushion. This visual characteristic may be observed and recorded by a camera over a specified time period. The recorded visual data is then analyzed by a processor to generate cardiac and/or respiratory information about the subject during the observation period.

This innovative approach offers several advantages over conventional methods. By combining the detection of both cardiac and respiratory signals into a single device, the system reduces the number of separate sensors required. The use of a camera to record visual changes eliminates the need for direct electrical connections to the subject, thereby reducing the complexity of the setup and minimizing potential interference with imaging equipment.

Furthermore, the system's ability to capture both cardiac and respiratory information from a single source may provide more comprehensive physiological data, potentially improving the accuracy of image gating and motion correction techniques in medical imaging procedures. The non-invasive nature of the sensor system may also enhance subject comfort during extended imaging sessions.

The amplification arrangement serves a critical function in the system by translating small pressure changes within the cushion into more easily detectable visual signals. This amplification allows for the capture of subtle physiological movements that might otherwise be difficult to measure accurately.

Indeed, it has been noticed that subtle pressure variations caused by the subject's heartbeat are discernable in addition to pressure variations caused by the subject's breath. However, these cardiac signals are much weaker than the respiratory component and have traditionally gone unused. Cardiac monitoring typically relies on separate systems like ECG leads or pulse oximeters, which require additional setup time and may cause subject discomfort. However, the system proposes to overcome this issue by amplifying the subtle pressure changes visually - both enabling the cardiac information to be discernable on top of the respiratory information in the pressure change and allowing measurement remotely by a camera.

An apparatus based on this sensor system concept may be adapted to provide indicators of cardiac and/or respiratory information. Such an apparatus comprises a cushion configured for compression by torso movement and an amplification arrangement with a visual characteristic dependent on the cushion's internal fluid pressure. This sensor system and apparatus represent a significant advancement in physiological monitoring technology for medical imaging applications. By simplifying the monitoring process while potentially improving data quality, this innovation may contribute to more efficient and effective medical imaging procedures.

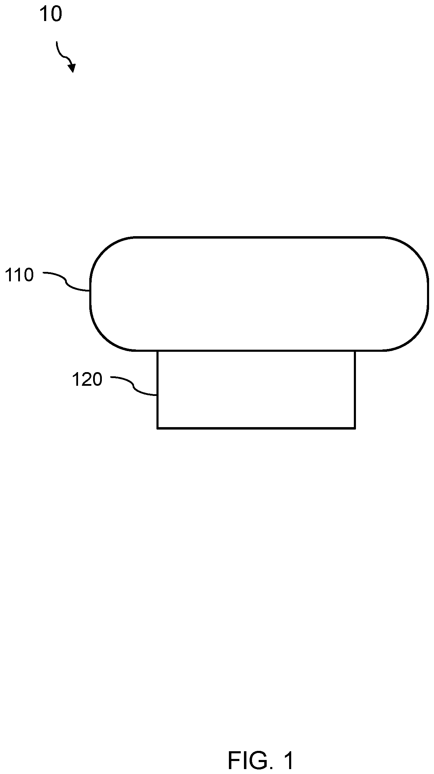

Referring to Fig. 1, there is presented a block diagram of an apparatus 10 adapted to provide an indicator of cardiac information and/or respiratory information of a subject according to aspects of the present disclosure.

The apparatus 10 includes a cushion component 110. The cushion component is configured to contain a fluid. The fluid may be a liquid (e.g., water) or a gas (e.g., air). Preferably, the fluid is a gas such that potential leaks due to perforation are avoided. Preferably, the fluid or gas is chosen as a material to minimize possible image artifacts on the image scanning device, e.g. MR imaging.

The cushion 110 may be any bag-type objection capable of containing the fluid, and which may have an internal fluid pressure that changes with compression. To this end, the cushion 110 may be formed of an elastic or semi-elastic material, and may be at least partially impermeable to the fluid within the cushion 110. The cushion 110 may further comprise contain a compressible solid medium, such that it better maintains its shape under compression and reverts back to an uncompressed state when external pressure is removed.

The cushion 110 is designed to respond to compression, which may be caused by movement of at least a part of a torso of a subject. As the cushion 110 is compressed, the fluid pressure within the cushion component 110 changes responsive to the compression. That is, if external pressure is applied to the cushion 110 (e.g., as a subject breathes in, or as blood vessels expand) the internal fluid pressure of the cushion 110 may increase. Once the external pressure is removed (e.g., the subject breathes out, or the blood vessel contracts) the internal fluid pressure of the cushion 110 may decrease.

To reiterate, the cushion 110 is configured to be compressible by movement of the torse of the subject. To this end, the cushion 110 may be positioned over the torso of the subject (and so may be shaped for this purpose). In some embodiments, the cushion 110 may be configured to be positioned proximate to the heart of the subject to enhance the capture of cardiac signals.

The apparatus 10 further comprises an amplification arrangement 120 which may be directly connected to and integrated with the cushion component 110. The amplification arrangement 120 is configured to exhibit visual characteristics based on pressure changes within the cushion component 110. These visual characteristics may include color changes, mechanical movements, or optical effects, depending on the specific implementation of the amplification arrangement 120.

In other words, the amplification arrangement 120 is configured to have a visual characteristic that visibly changes responsive to pressure changes in the cushion 110 that may arise due to movement of the torso of the subject that are attributable to respiratory and/or cardiac action. The amplification arrangement 120 may exacerbate these changes to be visually noticeable.

The amplification arrangement 120 may use various mechanisms to amplify and display pressure changes. These may include a pressure sensitive fluid that changes color intensity based on applied pressure, a spring-loaded gauge, a floating particle or membrane that changes position with pressure, an optical fiber that changes light wave guidance properties with pressure, or a point light source behind a mask with a small hole where the hole or light source moves laterally with cardiac motion.

The amplification arrangement 120 may incorporate various mechanisms to enhance and visualize pressure changes within the cushion. These mechanisms can be broadly categorized into mechanical, optical, and fluid-based systems, each offering unique advantages for different applications.

A mechanical amplification arrangement 110 may utilize structures that physically move or deform in response to pressure changes. For example, a diaphragm or membrane may be connected to a series of levers or gears, translating small movements into larger, more easily detectable displacements. In some cases, a spring-loaded piston system may be employed, where the compression of the spring correlates to the pressure within the cushion. These mechanical systems may offer high sensitivity and reliability, as well as the ability to function without electrical power.

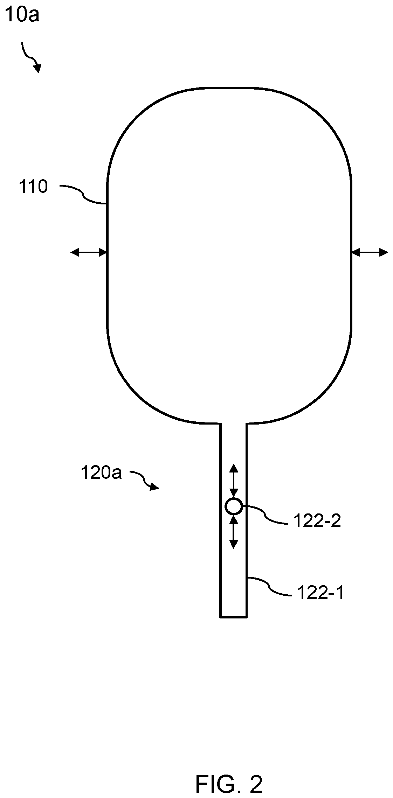

One such example is depicted in Fig. 2. This figure depicts an apparatus 10a comprising a cushion 110 that may be compressible by external pressure (as denoted by the arrows outside element 110). The apparatus 10a further comprises an amplification arrangement 120a in the form of a stem 122-1 having a diameter much smaller than the length or width of the cushion 110. The amplification arrangement 120a also comprises a small mechanical structure 122-2 or similar that may move in the stem 122-1 responsive to pressure changes in the cushion (as denoted by the arrows connected to the mechanical structure 122-2).

In other words, the stem portion 122-2 extends from the cushion body 110. The stem portion 122-1 includes a mechanical structure (e.g., a ball) 122-2 positioned along its length. The mechanical structure 122-2 is movable in a vertical direction as indicated by the bidirectional arrows adjacent to it. This mechanical structure may serve as part of the amplification arrangement 120, with its position dependent upon the fluid pressure within the cushion body 110.

That is, due to the relatively small size of the stem 122-1 compared to the cushion 110, a relatively small pressure change in the cushion 110 may result in the mechanical structure 122-2 moving a significant distance within the stem 122-1. The stem 122-1 may be at least partially transparent or may be partially expose to the surroundings such that movement of the mechanical structure 122-2 is visible. Thus, by movement of the ball 122-2, a pressure change within the cushion (which may be due to movement of the torso of the subject) may be derived.

Moving on, optical amplification arrangements may leverage the properties of light to visualize pressure changes. In some implementations, a flexible optical fiber may be integrated into the cushion, with its light transmission characteristics changing based on applied pressure. Alternatively, a system of mirrors or lenses may be used to create an optical lever, amplifying small movements into larger beam displacements. Some optical arrangements may utilize interference patterns or diffraction gratings, where pressure changes alter the observed light patterns. Optical systems may provide high precision and the ability to detect extremely small pressure variations.

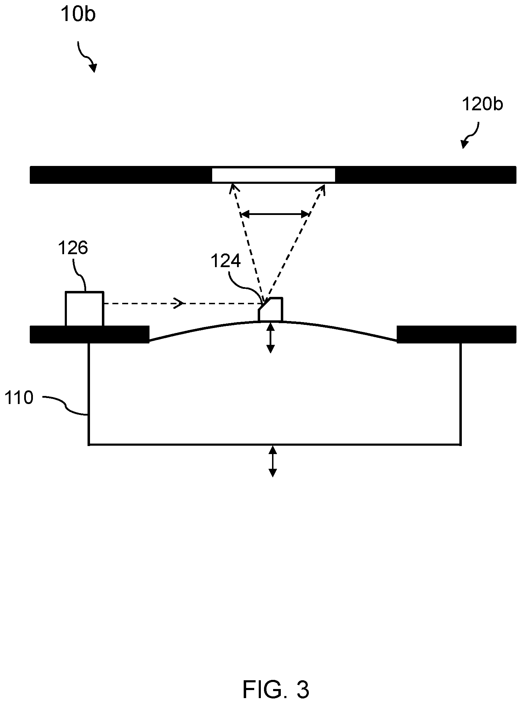

One such example is depicted in Fig. 3. This figure depicts an apparatus 10b comprising a cushion 110 that may be compressible by external pressure (as denoted by the arrow outside element 110). The apparatus 10b further comprises an amplification arrangement 120b including a reflective surface 124 positioned on the cushion.

The reflective surface 124 is mounted on a curved or flexible member that can deflect in response to pressure changes of the cushion. The position and/or orientation of the reflective surface 124 is thus dependent upon the fluid pressure within the cushion 110. A directional light source 126 is positioned on one side of the curved member and is configured to provide a beam of light incident to the reflective surface 124. As pressure changes cause the curved member to deflect, the angle of reflection changes, causing the reflected light to move across the light and dark portions of the upper structure.

Therefore, the resulting direction of the beam of light reflected by the reflective surface 124 represents the pressure in the cushion 110. This configuration allows small pressure variations to be amplified through the mechanical movement of the reflective surface 124 and corresponding changes in the reflected light pattern.

Moreover, fluid-based amplification arrangements 120 may employ pressure-sensitive fluids or gels that change their properties in response to applied pressure. For instance, some materials may change color or opacity when compressed, providing a visual indicator of pressure changes. Pressure-sensitive fluids are sometimes referred to as mechano-chromic or piezo-chromic fluids. One example of a pressure-sensitive fluid is a liquid crystal solution. One common type of liquid crystal solution that shifts color under pressure changes is cholesteric liquid crystals (ChLCs). ChLCs exhibit color properties due to their helical structure. Applying pressure distorts the helical structure of the fluid, thereby changing the reflected wavelength. This results in a visible color change which may be used as an indicator of applied pressure. In other cases, a system of interconnected fluid chambers may be used, where pressure changes in the main cushion are amplified through a series of smaller chambers. Fluid-based systems may offer smooth, continuous responses to pressure changes and can be designed to be highly sensitive to specific pressure ranges.

Some amplification arrangements 120 may combine elements from multiple categories. For example, a hybrid system might use a mechanical diaphragm to compress a small amount of pressure-sensitive fluid, which then alters the path of a light beam. This type of multi-stage amplification may allow for fine-tuning of the system's sensitivity and response characteristics.

The choice of amplification arrangement 120 may depend on factors such as the required sensitivity, the expected range of pressure changes, the imaging environment, and the specific physiological signals being monitored. For instance, in MRI applications, non-magnetic materials may be crucial, favoring optical or certain fluid-based systems. In situations where rapid pressure changes need to be detected, mechanical or optical systems with fast response times may be preferred.

Some amplification arrangements 120 may be designed to selectively amplify certain frequency ranges, potentially allowing for better separation of cardiac and respiratory signals. For example, a mechanical system with specific resonant frequencies could be tuned to emphasize either the faster cardiac rhythms or the slower respiratory cycles.

The design of the amplification arrangement 120 may also consider factors such as durability, ease of cleaning and sterilization, and compatibility with different subject sizes and body shapes. Some arrangements may be modular or adjustable, allowing for customization based on the specific clinical application or subject characteristics.

By carefully selecting and optimizing the amplification arrangement 120, the apparatus 10 may achieve high sensitivity to physiological movements while maintaining robustness and reliability in clinical settings. The versatility of possible amplification mechanisms allows for the development of specialized systems tailored to specific medical imaging and subject monitoring scenarios.

Finally, the apparatus 10 may include a biasing means configured to pressurize the cushion 110 toward the torso of the subject. This may be in the form of a weight that provides constant pressure between the torso and the cushion 110. In some examples, the biasing means may comprise a magnetic resonance (MR) coil, which may integrate the apparatus seamlessly with an MR imaging application. In this case, the amplification arrangement 120 may be at least partially integrated with the MR coil.

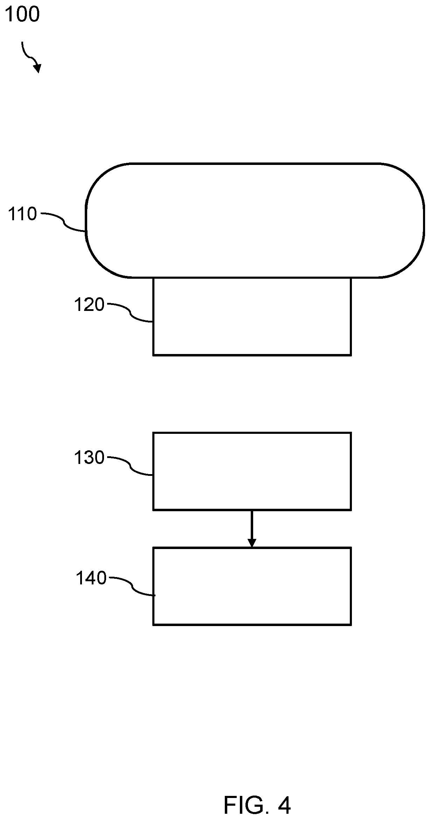

Referring to Fig. 4, a block diagram of a sensor system 100 is illustrated. The system includes the cushion 110 positioned at the top of the diagram, which contains a fluid and is configured for compression. The system also comprises the amplification arrangement 120 that exhibits visual characteristics based on fluid pressure changes within the cushion 110. Repeated description of these elements is omitted here for sake of brevity.

The system further includes a camera 130. The camera 130 is configured to record the visual characteristics of the amplification arrangement 120 over a time period. These visual characteristics may include color changes of a pressure sensitive fluid, the position and/or shape of a mechanical structure, or the path of light provided by a light source, depending on the specific implementation of the amplification arrangement 120.

The camera 130 may be positioned so as to be able to capture the visual characteristic of the amplification arrangement. The camera 130 may be fixed in-bore, semi-attached to a gantry, or disjunct from the MRI or CT scanner or similar system, depending on the specific implementation and requirements of the imaging system. Of course, there may be multiple cameras 130 provided so that the amplification arrangement 120 is always visible regardless of positioning of the apparatus on the subject.

A processor 140 is communicatively coupled to the camera 130. The processor 140 is configured to analyze the recorded visual characteristics and generate, based on the analysis, cardiac information and/or respiratory information of the subject during the time period. To be clear, the cardiac information may simply comprise a heart rate, a time at which heart beats occur, or may comprise more granular information such as heart rate variability and/or the full cardiac signal. The respirator information may simply comprise a respiration rate or a time at which breath holds occur, or may comprise more granular information such as respiration rate variability and/or the full respiration signal.

The processor 140 may be configured to analyze the recorded visual characteristic to determine pressure changes within the cushion 110 over the time period and generate the cardiac information and/or the respiratory information based on the determined pressure changes. To this end, the processor 140 may utilize image recognition techniques to extract this information from the data captured by the camera, and other algorithms. The processor may utilize algorithms suitable for translating the changes of the visual characteristic into a pressure change, and other algorithms suitable for generating cardiac information and/or the respiratory information from the determined pressure change.

To be clear, the processor may not generate an absolute measurement of the pressure within the cushion 110, but may instead generate a relative measurement of the pressure from which cardiac and/or respiratory information may be derived. Indeed, it may not be necessary to determine the absolute pressure within the cushion 110 to derive this information.

In some examples, the processor 140 may be configured to receive external respiratory information from a further sensor. The external respiratory information may describe respiration of the subject during the time period. The processor 140 may then analyze the recorded visual characteristic and the respiratory information to generate the cardiac information.

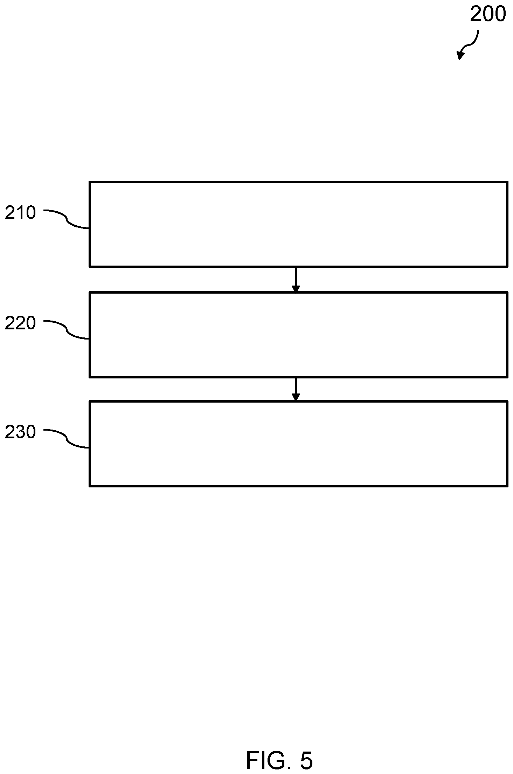

Fig. 5 illustrates a flowchart depicting a method 200 for generating cardiac and/or respiratory information, according to aspects of the present disclosure.

In step 210, a cushion is compressed by movement of at least a part of a torso of a subject. The cushion may be any cushion as disclosed herein, wherein fluid pressure within the cushion changes responsive to compression of the cushion.

In step 220, a visual characteristic of an amplification arrangement over a time period is recorded (i.e., measured or detected). As outlined above, the visual characteristic of the amplification arrangement is dependent upon the fluid pressure within the cushion.

Finally, in step 230 the recorded visual characteristic is analyzed to generate cardiac information and/or respiratory information of the subject during the time period.

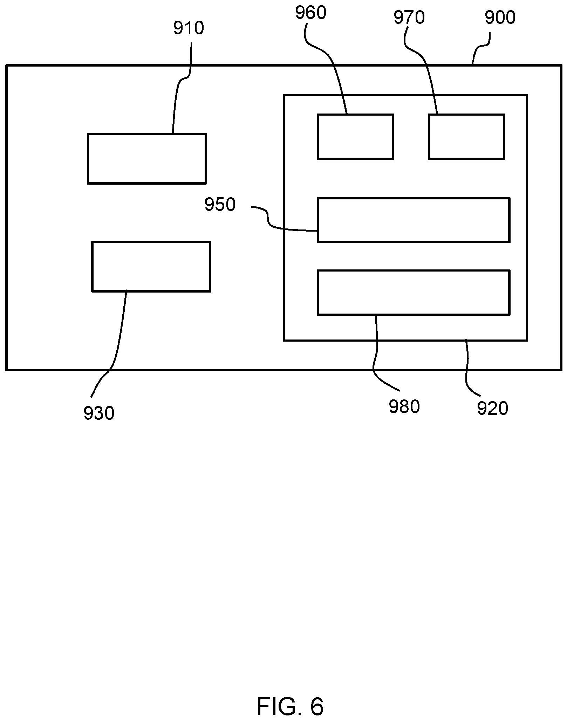

Fig. 6 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.
The I/O devices 930 may include input devices such as, for example but not limited to, a mouse, keyboard, scanner, microphone, camera, etc. Furthermore, the I/O devices 930 may also include output devices, for example but not limited to a printer, display, etc.

Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to provide an advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A sensor system (100), comprising:
a cushion (110) configured for compression by movement of at least a part of a torso of a subject, the cushion containing a fluid, and wherein a fluid pressure within the cushion changes responsive to compression of the cushion;
an amplification arrangement (120) having a visual characteristic dependent upon the fluid pressure within the cushion;
a camera (130) configured to record the visual characteristic of the amplification arrangement over a time period; and
a processor (140) configured to analyse the recorded visual characteristic, and to generate, based on the analysis, cardiac information and/or respiratory information of the subject during the time period.

2. The system of claim 1, wherein the amplification arrangement (120) comprises a pressure sensitive fluid having a colour characteristic dependent upon the fluid pressure within the cushion (110), and the camera (130) is configured to record the colour characteristic of the pressure sensitive fluid over the time period.

3. The system of claim 1 or 2, wherein the amplification arrangement (120a) comprises a mechanical structure (122) having a position and/or shape dependent upon the fluid pressure within the cushion (110), and the camera (130) is configured to record the position and/or shape of the mechanical structure over the time period.

4. The sensor of any of claims 1 to 3, wherein the amplification arrangement (120) comprises:
an optical structure having a light wave guidance property dependent upon the fluid pressure within the cushion (110); and
a light source configured to provide light to the optical structure, and
wherein the camera (130) is configured to record at least a part of the path of the light provided by the light source.

5. The system of any of claims 1 to 4, wherein the amplification arrangement (120b) comprises:
a reflective surface (124) having a position and/or orientation dependent upon the fluid pressure within the cushion (110); and
a directional light source (126) configured to provide a beam of light incident to the reflective surface, and
wherein the camera (130) is configured to record a resulting direction of the beam of light reflected by the reflective surface.

6. The system of any of claims 1 to 5, further comprising a biasing means configured to pressurize the cushion (110) toward the torso of the subject.

7. The system of claim 6, wherein the biasing means comprises a magnetic resonance, MR, coil, and optionally wherein the amplification arrangement (120) is at least partially integrated with the MR coil.

8. The system of any of claims 1 to 7, wherein the cushion (110) further contains a compressible solid medium.

9. The system of any of claims 1 to 8, wherein the cushion (110) is configured to be positioned proximate to the heart of the subject.

10. The system of any of claims 1 to 9, wherein the processor (140) is configured to:
analyse the recorded visual characteristic to determine pressure changes within the cushion (110) over the time period; and
generate the cardiac information and/or the respiratory information based on the determined pressure changes.

11. The system of any of claims 1 to 10, wherein the processor (140) is configured to:
receive external respiratory information from a further sensor, the external respiratory information describing respiration of the subject during the time period; and
analyse the recorded visual characteristic and the respiratory information, and to generate, based on the analysis, the cardiac information.

12. The system of any of claims 1 to 11, wherein the cardiac information comprises a cardiac signal, a heart rate, and/or a heart rate variability, and wherein the respiratory information comprises a respiratory signal, a respiratory rate and/or breath hold timings.

13. A method (200) for generating cardiac information and/or respiratory information of a subject, comprising:
compressing (210) a cushion by movement of at least a part of a torso of a subject,
wherein fluid pressure within the cushion changes responsive to compression of the cushion;
recording (220) a visual characteristic of an amplification arrangement over a time period, wherein the visual characteristic of the amplification arrangement is dependent upon the fluid pressure within the cushion; and
analysing (230) the recorded visual characteristic to generate cardiac information and/or respiratory information of the subject during the time period.

14. A computer program comprising computer program code means adapted, when said computer program is run on a computer, to implement the method of claim 13.

15. An apparatus (10) adapted to provide an indicator of cardiac information and/or respiratory information of a subject, the apparatus comprising:
a cushion (100) configured for compression by movement of at least a part of a torso of a subject, the cushion containing a fluid, and wherein a fluid pressure within the cushion changes responsive to compression of the cushion; and
an amplification arrangement (200) having a visual characteristic dependent upon the fluid pressure within the cushion.
